Europäisches Patentamt

European Patent Office

Office européen des brevets

(11) Veröffentlichungsnummer: **0 219 606 B1**

(12) **EUROPÄISCHE PATENTSCHRIFT**

(45) Veröffentlichungstag der Patentschrift:
11.04.90

(51) Int. Cl.⁴: **A61N 1/36**

(21) Anmeldenummer: **86107948.1**

(22) Anmeldetag: **11.06.86**

(54) **Herzschrittmacher.**

(30) Priorität: **02.10.85 DE 3535181**

(43) Veröffentlichungstag der Anmeldung:
**29.04.87 Patentblatt 87/18**

(45) Bekanntmachung des Hinweises auf die Patenterteilung:
**11.04.90 Patentblatt 90/15**

(84) Benannte Vertragsstaaten:
**DE FR GB IT NL SE**

(56) Entgegenhaltungen:
**US-A- 3 599 627**
**US-A- 3 618 615**
**US-A- 4 231 027**

(73) Patentinhaber: **Siemens Aktiengesellschaft, Wittelsbacherplatz 2, D-8000 München 2(DE)**

(84) Benannte Vertragsstaaten: **DE FR GB IT NL SE**

(73) Patentinhaber: **Siemens Elema AB, Röntgenvägen 2, S-171 95 Solna 1(SE)**

(84) Benannte Vertragsstaaten: **SE**

(72) Erfinder: **Moberg, Lennart, Dipl.-Ing., Kastrupgatan 3, S-163 42 Spanga(SE)**

ACTORUM AG

## Beschreibung

Die Erfindung betrifft einen Herzschrittmacher mit einer Testeinheit, die ein dem Ladezustand der Batterie entsprechendes elektrisches Signal liefert.

Es ist bekannt, den Ladezustand der Batterie eines Herzschrittmachers beim Test dadurch anzuzeigen, daß Testimpulse mit davon abhängiger Frequenz abgegeben werden. Diese Frequenz kann über das EKG ermittelt werden. Dies bedeutet, daß während des Testbetriebes die therapeutisch eingestellte Stimulierungsfrequenz geändert werden muß.

Der Erfindung liegt die Aufgabe zugrunde, einen Herzschrittmacher der eingangs genannten Art so auszubilden, daß eine einfache analoge Anzeige des Ladezustandes der Batterie erfolgt, ohne daß die Frequenz der Stimulationsimpulse dadurch beeinflußt wird.

Diese Aufgabe ist erfindungsgemäß dadurch gelöst, daß das dem Ladezustand der Batterie entsprechende elektrische Signal einen Pulsgenerator steuert, welcher mindestens einen Anzeigeimpuls am Ausgang des Herzschrittmachers erzeugt, dessen zeitliche Lage in bezug auf die Stimulationsimpulse vom jeweiligen Ladezustand der Batterie abhängt. Bei dem erfindungsgemäßen Herzschrittmacher ist ein Ablesen des Ladezustandes der Batterie aus dem EKG möglich, ohne daß die normale Stimulierung des Herzens gestört wird. Die Anzeige des Ladezustandes kann dabei auf folgende Arten erfolgen:

a) Der Markierungsimpuls wird in jedem Intervall zwischen zwei Stimulationsimpulsen abgegeben, solange der Herzschrittmacher zum Auslesen des Ladezustandes der Batterie aktiviert ist. Die Position des Markierungsimpulses in bezug auf den vorhergehenden oder nachfolgenden Stimulationsimpuls gibt den Ladezustand an.

b) In jedem n-ten Intervall wird ein Markierungsimpuls abgegeben, dessen Lage vom Ladezustand der Batterie abhängt. n ist dabei eine ganze Zahl und die Lage des Markierungsimpulses im Intervall ergibt die Kommastelle nach dieser ganzen Zahl. Dadurch ist eine hohe Anzeigegenauigkeit gegeben.

Die Erfindung ist nachfolgend anhand von in der Zeichnung dargestellten Ausführungsbeispielen näher erläutert. Es zeigen:

Fig. 1 und 2 zwei verschiedene Ausführungsbeispiele zur Bestimmung des Ladezustandes der Batterie eines Herzschrittmachers,

Fig. 3 einen Impulsverlauf zur Anzeige des Ladezustandes,

Fig. 4 eine Schaltungsanordnung zur Erzeugung des Impulsverlaufes gemäß Figur 3,

Fig. 5 eine Schaltung zur Erzeugung eines anderen Impulsverlaufes für die Anzeige des Ladezustandes der Batterie,

Fig. 6 einen durch die Schaltung gemäß Figur 5 erzeugten Impulsverlauf, und

Fig. 7 eine Schaltung zur Erzeugung des Impulsverlaufes gemäß Figur 6.

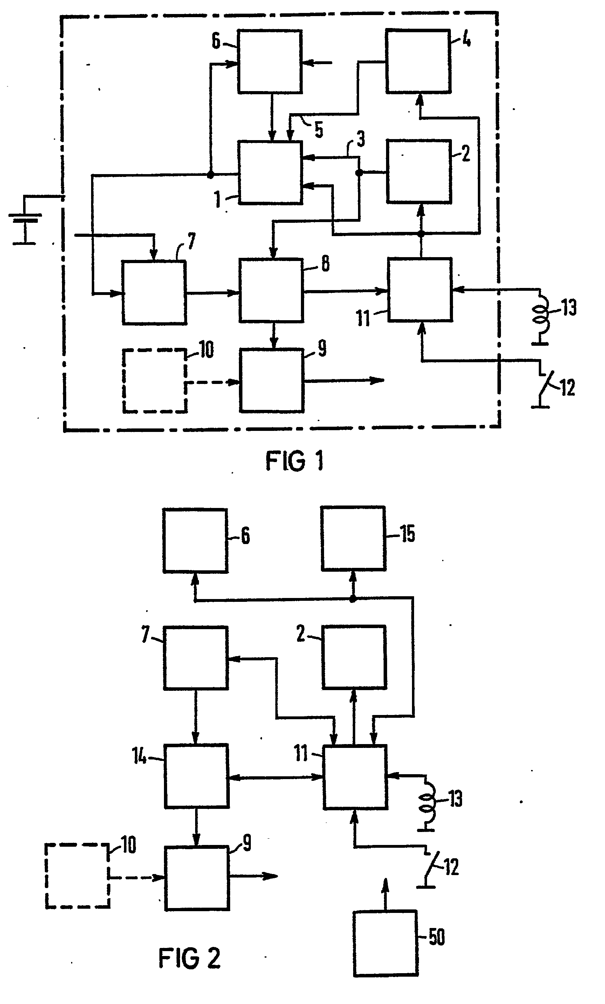

Bei der Schaltungsanordnung gemäß Figur 1, die insgesamt in einen Herzschrittmacher integrierbar ist, wird einer Logik 1 von einem Moderegister 2 am Eingang 3 ein Signal zugeführt, das den eingestellten Werten des Herzschrittmachers entspricht. Ein Impedanzregister 4 liefert am Eingang 5 ein der Elektrodenimpedanz entsprechendes Signal. Die seit dem letzten Test erzeugten Stimulationsimpulse werden in einem Stimulationspulszähler 6 gezählt. Ein Ladungszähler 7 dient zur Speicherung eines dem Ladezustand der Batterie entsprechenden elektrischen Signales, das über eine Logik 8 eine Ausgangsstufe 9 ansteuert, die an der Schrittmacherlogik 10 angeschlossen ist. Die Ansteuerung der Schaltungsanordnung erfolgt durch einen Empfänger 11 über einen magnetisch betätigbaren Kontakt 12 oder mittels eines Senders/Empfängers drahtlos über eine Spule 13.

Wird die Schaltungsanordnung gemäß Figur 1 durch den Sender/Empfänger 11 aktiviert (Test-Betrieb), so werden die Werte des Herzschrittmachers, wie die eingestellten Parameter für die Impulsamplitude, die Impulsdauer und die Stimulationsfrequenz zur Logik 1 überführt. Die Logik 1 berechnet daraus, aus der Elektrodenimpedanz und unter Berücksichtigung eines Grundverbrauches, die jeweils verbrauchte Ladung, z.B. ausgedrückt in Grundeinheiten von 5 µAh. Hierzu werden die seit dem letzten Test aufgelaufenen Stimulationsimpulse, die der Stimulationspulszähler 6 der Logik 1 meldet, berücksichtigt. Die verbrauchte Ladung wird im Ladungszähler 7 aufsummiert. Der Inhalt des Ladungszählers 7 ist demgemäß ein Maß für die jeweils verbrauchte Ladung und wird über die Logik 8 und die Ausgangsstufe 9 in das Ausgangssignal des Schrittmachers eingeblendet, wie dies nachfolgend näher beschrieben ist.

Bei der Schaltungsanordnung gemäß Figur 2 ist wieder ein Sender/Empfänger 11 vorhanden, der durch einen Reed-Schalter 12 oder eine Spule 13 aktivierbar ist. Teile, die mit Teilen der Figur 1 gleich sind, sind mit gleichen Bezugszeichen bezeichnet. Zusätzlich zur Figur 1 ist ein Zeitregister 15 vorhanden. Die Komponenten 2, 6, 7 sind anders zusammengeschaltet als bei dem Ausführungsbeispiel gemäß Figur 1.

Beim Testbetrieb liest eine externe Einheit 50 zunächst das Zeitregister 15. Dieses Zeitregister 15 liefert ein dem jeweiligen Zeitpunkt der Messung entsprechendes elektrisches Signal im Rahmen einer vorbestimmten Genauigkeit von z.B. zwei Stunden. Dies bedeutet, daß alle zwei Stunden ein neues Signal vom Zeitregister 15 erzeugt wird.

Mit Kenntnis des Meßzeitpunktes ist die externe Einheit in der Lage, den Grundverbrauch zu bestimmen. Danach liest die externe Einheit den Wert des Stimulationspulszählers 6 aus, der die Anzahl der Stimulationsimpulse seit dem letzten Batterietest enthält. Wenn der Stimulationspulszähler 6 Null anzeigt, so wird der Ladungszähler 7 allein mit dem Grundverbrauch beaufschlagt. Wenn der Stimulationspulszähler 6 einen von Null verschiedenen Wert enthält, führt die externe Einheit folgende Schritte aus:

Zunächst werden Register für die Impulsamplitude, die Impulsdauer und die Stimulationsfrequenz abgelesen. Danach berechnet die externe Einheit die Elektrodenimpedanz. Mit Kenntnis dieser Werte kann die externe Einheit über eine Tabelle den Ladungsverbrauch jedes Stimulationsimpulses berechnen. Dieser Verbrauch wird mit der Anzahl der Stimulationsimpulse im Stimulationspulszähler 6 multipliziert und zum Grundverbrauch addiert. Danach wird der Ladungszähler 7 mit diesem Wert beaufschlagt und der Stimulationspulszähler 6 auf Null zurückgesetzt.

Bei Umprogrammierungen, die den Verbrauch beeinflussen, wird jedesmal ein Batterietest durchgeführt.

Für die Bildung eines der verbleibenden Batteriekapazität entsprechenden elektrischen Signales wandelt die Logik 14 den Wert des Ladungszählers 7 in eine verbleibende Zeit um. Diese Zeit kann über den Sender/Empfänger 11 ausgelesen werden. Es ist aber auch möglich, ein entsprechendes Signal in der beschriebenen Weise über die Ausgangsstufe 9 einzublenden.

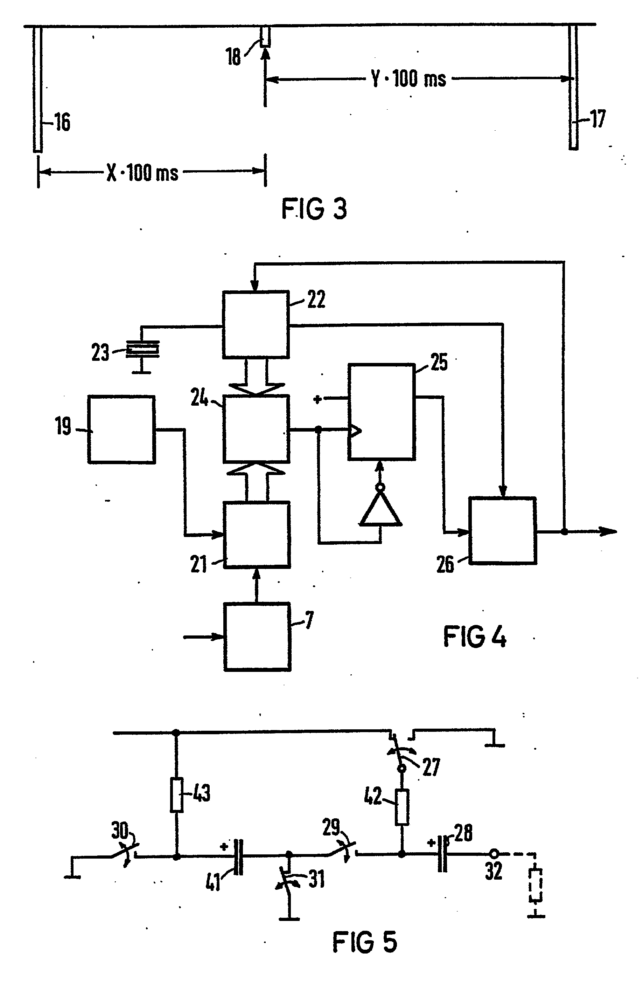

Die Figur 3 zeigt zwei aufeinanderfolgende Stimulationsimpulse 16, 17 eines Herzschrittmachers und einen eingeblendeten Markierungsimpuls 18. Der Markierungsimpuls 18 wird von der Ausgangsstufe 9 erzeugt. Der zeitliche Abstand des Markierungsimpulses 18 vom Stimulationsimpulse 16 hängt dabei von der noch vorhandenen Restkapazität der Batterie ab und beträgt bei dem Beispiel x mal 100 ms oder y mal 100 ms. x oder y drückt dabei die verbleibende Batteriekapazität in Jahren aus. Der Markierungsimpuls 18 wird nur im Testbetrieb eingeblendet. Während dieser Zeit arbeitet der Herzschrittmacher mit fester Frequenz.

Die Figur 4 zeigt eine Schaltungsanordnung 19 zur Aktivierung des Batterietestes, die eine Logik 21 ansteuert. Der Logik 21 wird der Inhalt des Ladungszählers 7 zugeführt. Er wird hier in eine Zeit umgewandelt, die der verbleibenden Kapazität in Jahren (x oder y) entspricht. Dieser Wert x (y) wird in einen Abstand des Markierungsimpulses 18 vom Stimulationsimpulse 16 bzw. 17 umgesetzt, der x (y) mal 100 ms beträgt. Durch einen Vergleich des Wertes in der Logik 21 mit einer Zeitbasis, die von einem Zeitgenerator 22 mit einem Schwinger 23 geliefert wird, in einem Vergleicher 24 wird die korrekte Position des Markierungsimpulses 18 festgelegt, der über eine Steuerstufe 25 und eine Ausgangsstufe 26 in die Ausgangsimpulse des Herzschrittmachers eingeblendet wird.

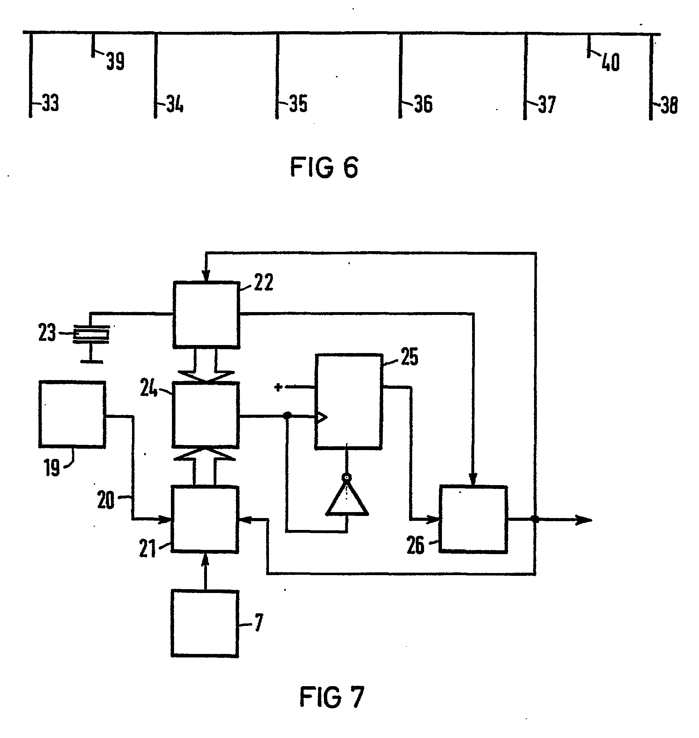

Die Figur 5 zeigt die Ausgangsstufe der Schaltung gemäß Figur 4. Bei Normalbetrieb nimmt ein Schalter 27 die gezeichnete Position ein. Während der Aufladungsphase eines Ausgangskondensators 28 sind Schalter 29, 30 geöffnet und 31 geschlossen. Für die Erzeugung eines Ausgangsimpulses werden die Schalter 29, 30 geschlossen sowie der Schalter 31, der vorher geschlossen war, geöffnet. Dadurch wird am Ausgang 32 ein Stimulationsimpuls erzeugt.

Während des Testzustandes arbeitet der Herzschrittmacher, wie beschrieben, mit einer festen Frequenz. Wenn der Wert in der Logik 21 (Fig. 4) mit dem Wert der Zeitbasis 22 gleich ist, wird der Schalter 27 kurzzeitig, z.B. während 1 ms umgeschaltet. Die Schaltung gemäß Figur 5 ist so bemessen, daß die Amplitude des dadurch erzeugten Markierungsimpulses nicht ausreicht, um das Herz zu stimulieren.

Bei der Impulsfolge gemäß Figur 6 liefert der Herzschrittmacher während des Testbetriebes Stimulationsimpulse 33 bis 38 mit einer festen Frequenz. Es werden Markierungsimpulse 39, 40 usw. erzeugt, die x mal 100 ms nach den vorhergehenden Stimulationsimpulsen 33, 37 oder y mal 100 ms vor dem nachfolgenden Stimulationsimpuls 34, 38 usw. liegen und bei denen der Markierungsimpuls 40 im n-ten Intervall nach dem vorhergehenden Markierungsimpuls 33 auftritt. Dies bedeutet, daß bei n = 7 und x = 4 (y = 4) die Restkapazität der Batterie größer als 7,4 Jahre ist.

Die Erzeugung der Markierungsimpulse 39, 40 erfolgt mit der Schaltung gemäß Figur 7, die weitgehend mit der Schaltung gemäß Figur 4 übereinstimmt, so daß gleiche Teile wieder mit gleichen Bezugszeichen bezeichnet sind.

Beim Batterietest wird der Wert des Ladungszählers 7 in eine Anzahl von Intervallen umgewandelt, die durchlaufen werden müssen, bevor der Markierungsimpuls 40 erzeugt wird. Wenn das richtige Intervall kommt, wird der Markierungsimpuls 39 zunächst in der richtigen Lage im Intervall erzeugt. Hierzu findet eine Ausgangsstufe Verwendung, wie sie in der Figur 5 bereits dargestellt ist. In dieser Ausgangsstufe ist der Schalter 27 stets in der gezeigten Stellung, wenn kein Batterietest erfolgt. Während der Aufladungsphase des Ausgangskondensators 28 sind die Schalter 29 und 30 geöffnet und der Schalter 31 geschlossen. Die Kondensatoren 28 und 41 werden über Widerstände 42 und 43 aufgeladen.

Beim Markierungsimpuls werden die Schalter 29 und 30 geschlossen und der Schalter 31 geöffnet. Die Ausgangsamplitude ist wieder so gewählt, daß keine Stimulation des Herzens erfolgt und die Herzschrittmacherfrequenz ist während des Testbetriebes fest. Der Wert der Logik 21 wird mit der Zeitbasis verglichen. Wenn die Werte gleich sind, also wenn das richtige Zeitintervall auftritt und die richtige Anzahl von Stimulationsimpulsen seit dem Markierungsimpuls 39 in Figur 6 aufgetreten ist, wird durch den Schalter 27 der Markierungsimpuls 40 erzeugt. Auch dieser Markierungsimpuls stimuliert das Herz nicht.

## Patentansprüche

1. Herzschrittmacher mit einer Testeinheit (Fig. 1, 2), die ein dem Ladezustand der Batterie entsprechendes elektrisches Signal liefert, **dadurch gekennzeichnet**, daß dieses Signal einen Pulsgenerator (Fig. 4, 5, 7) steuert, welcher mindestens einen Anzeigeimpuls (18, 39, 40) am Ausgang des Herzschrittmachers erzeugt, dessen zeitliche Lage in bezug auf die Stimulationsimpulse (16, 17, 33 bis 38) vom jeweiligen Ladezustand der Batterie abhängt.

## Claims

1. A heart pacemaker with a test unit (Figures 1, 2), which delivers an electric signal corresponding to the charging state of the battery, characterised in that this signal controls a pulse generator (Figures 4, 5, 7), which generates at least one display pulse (18, 39, 40) at the output of the heart pacemaker, the temporal position of which, relative to the stimulation pulses (16,17, 33 to 38) depends on the respective charging state of the battery.

## Revendications

1. Stimulateur cardiaque comportant une unité d'essai (figures 1, 2), qui délivre un signal électrique correspondant à l'état de charge de la pile, caractérisé par le fait que ce signal commande un générateur d'impulsions (figures 4, 5, 7), qui produit, à la sortie du stimulateur cardiaque, au moins une impulsion d'affichage (18, 39, 40), dont la position dans le temps par rapport aux impulsions de stimulation (16, 17, 33 à 38) dépend de l'état de charge respectif de la pile.

EP 0 219 606 B1

FIG 1

FIG 2

FIG 3

FIG 4

FIG 5

**FIG 6**

**FIG 7**